# EUROPÄISCHE PATENTSCHRIFT

(11) **EP 0 776 184 B1**
(45) Veröffentlichungstag und Bekanntmachung des Hinweises auf die Patenterteilung: **29.04.1998**
(21) Anmeldenummer: 94926173.9
(22) Anmeldetag: 10.08.1994
(51) Int. Cl.: A61F 5/058, A61F 5/01

(54) **HAND- UND HANDGELENKORTHESE**
HAND AND WRIST ORTHOSIS
ORTHESE POUR MAIN ET POIGNET

(30) Priorität: 11.08.1993 DE 4326751
(43) Veröffentlichungstag der Anmeldung: 04.06.1997
(73) Patentinhaber: Beiersdorf Aktiengesellschaft, 20245 Hamburg (DE)
(72) Erfinder: BRANDT, Dieter, D-40545 Düsseldorf (DE); SZLEMA, M., A., Ingeborg, D-47906 Kempen (DE)
(74) Vertreter: Richter, Joachim, Dipl.-Ing.
(86) Internationale Anmeldenummer: EP9402655
(87) Internationale Veröffentlichungsnummer: WO9504507

(56) Entgegenhaltungen:
- DE-U- 9 112 665
- US-A- 4 716 892
- US-A- 5 058 576
- US-A- 5 152 739

## Beschreibung

Die Erfindung betrifft eine Hand- und Handgelenkorthese, insbesondere zur Behandlung von Tendovaginitis, die aus einem an der Hand und an dem Handgelenk mittels Verschlußbändern gehaltenen, formgenähten, Stützkörper mit einer volaren, die Hohlhand abstützenden, starren Schiene besteht, um die Hand und die Finger in Funktionsstellung zu halten, und die mit einem fingerzugewandten Abschnitt gelenkübergreifend im Bereich der Fingerglieder endet und sich über den größten Teil der Finger erstreckt.

Eine Hand- und Handgelenkorthese beschreibt die US-A-5,152,739. Diese Orthese besteht aus einem schalenförmigen Stützkörper, der gleichzeitig die volare Schiene bildet und die zur Abwinklung der Hand einendseitig mit einer schräg verlaufenden Wandfläche versehen ist, an die sich zwei abgewinkelte Abschnitte zur Fingerfixierung anschließen, so daß im Bereich der Fingerglieder und der Mittelhandknochen drei zueinander abgebogene Schienenabschnitte mit unterschiedlichen Winkelstellungen ausgebildet werden, wobei jedoch alle Knicklinien quer zur Schienenlängsrichtung verlaufen, mit der jedoch die medizinische Funktionsstellung nut annähernd erreicht wird, weil der Knicklinienverlauf der zueinander abgewinkelten Schienenabschnitte nicht den Gelenklinien den durch die Fingergliedergelenke verlaufenden Gelenklinien entspricht, da diese bis auf die im Bereich des Handgelenkes quer zur Handlängsrichtung verlaufende Gelenklinie schräg zur Handlängsrichtung verlaufen.

Die US-A-4,716,892 beschreibt eine Hand- und Handgelenkorthese aus einem bandagenartigen Stützkörper, der mittels Verschlußbändern an der Hand und an dem Handgelenk festlegbar ist und in dem eine volare Schiene eingearbeitet ist, um die Hand in Funktionsstellung zu halten, insbesondere zur Behandlung von Tendovaginitis, wobei die Orthese aus einem formgenähten Handgelenk und Hand hüllenartig umschließenden Stützkörper, der mit einem fingerzugewandten Abschnitt gelenkübergreifend im Bereich der Fingerglieder endet und sich über den größten Teil der Finger erstreckt, und aus einer volarartig an der Innenwandfläche des Stützkörpers angeordneten löffenförmigen, die Hohlhand abstützenden und den Spitzgriff ermöglichenden starren Schiene besteht, die jedoch keine im Bereich der Fingerglieder und der Mittelhandknochen zueinander abgebogenen Schienenabschnitte aufweist. Diese Orthese stellt eine dynamische Handschiene dar, um die Hand so zu fixieren, daß eine medizinische Handfunktionsstellung unter Ausschluß der Finger erreicht wird, denn alle Finger der Hand bleiben freibeweglich, denn es erfolgt keine Vorgabe einer leichten Beugestellung im Handgelenk und den Fingergelenken.

Durch DE-U-17 61 705 ist eine Vorrichtung zur Ruhigstellung der menschlichen Hand und Finger bekannt, bei der die Verbindung einer Schiene mit einem Körper erfolgt, dessen Formgebung so beschaffen ist, daß die Hand bzw. die jeweils ruhig zu stellenden Finger in Funktionsstellung auf diesem Körper ruhen können, und daß ferner der Körper mit Durchbrechungen, z. B. Schlitzen, ausgestattet ist, welche nötigenfalls die Anbringung von besonderen Festhaltemitteln, Polstern usw. sowie einen Luftzutritt gestatten. Es wird hiernach eine Schiene mit einem Körper verbunden, wobei die Schiene eine Formgebung aufweist und als dynamische Handschiene die Hand in der medizinischen Funktionsstellung hält. Wird dem Körper, der mit der Schiene zu einer Einheit verbunden ist, die Gestalt einer vorbestimmbaren Raumkurve, deren Querwölbung der Hohlhand in Höhe der Fingergrundgelenke und dem kovergierenden Verlauf der Finger beim Faustschluß gerecht wird und deren Längswölbung so gewählt ist, daß die Finger wie Tangenten der gewölbten Hand anliegen, wobei die Fingergelenke zwangsläufig in die gewünschte Gelenkstellung gebracht werden, so läßt sich durch einfaches Anwinkeln der Schiene eine Ruhigstellung in Funktionsstellung erreichen. Die Formgebung und Größe des Körpers der Schiene wird entsprechend einem Mittelwert der in der Natur anzutreffenden Größenordnung der Hand von Erwachsenen gewählt, so daß eine Einheitsvorrichtung zum Schienen der Hand erzielt wird, die für nahezu alle Hände und Finger passen soll. Auftretende geringe Größendifferenzen der verschiedenen Hände sollen dabei dadurch ausgeglichen werden, daß die jeweilige Hand etwas mehr oder weniger die Fläche der Vorrichtung umgreift. Dadurch ist es jedoch nicht möglich, die Grundgelenke der Langfinger in 70° bis 80° Flexion zu lagern und zu fixieren, da keine Paßgenauigkeit gegeben ist. Hinzu kommt, daß der an dem einen Ende der Schiene ausgebildete Stützkörper als Hohlkörper ausgebildet ist, so daß bei angelegter Schiene die Langfinger der Hand diesen Hohlkörper umgreifen, der einen etwa kreisförmigen Querschnitt aufweist. Dadurch ist eine eindeutige Winkelfestlegung der Gelenke der Finger zueinander nicht gewährleistet. Die Befestigung der Schiene am Unterarm, Handgelenk und Finger erfolgt durch Anlegen einer Bandage. Um einen oder mehrere Langfinger der Hand zu fixieren, bedarf es einer komplizierten Bandagenführung. Das Anlegen dieser Handschiene ist daher nur durch geschultes Fachpersonal möglich. Bei nicht einwandfreier, druckentlastender Führung der Bandage beim Anlegen und Fixieren der Handschiene kann es somit zu Einschnürungen und damit zu Blutstauungen kommen. Auch ist für einen längeren Zeitraum keine stabile Fixierung möglich, was auf das Nachlassen der Zugkräfte der Bandagen zurückzuführen ist.

Das DE-U-81 28 832 beschreibt eine elastische Handgelenksbandage zur wahlweisen Anwendung als elastische Stütze oder zur Stillegung des Handgelenks; sie ist so ausgebildet, daß an der Innenhand eine herausnehmbare Schiene vorgesehen ist. Bei dieser Handgelenksbandage handelt es sich jedoch in keiner Weise um eine dynamische Handschiene zur Lagerung der Hand in der bekannten medizinischen Funktionsstellung. Die Langfinger der fixierten Hand sind bei angelegter Handgelenksbandage frei beweglich. Die Schiene erstreckt sich über einen Abschnitt des Unterarms über das Handgelenk bis in die Innenhand und hat ausschließlich Stützungsfunktion.

Eine manschettenartig ausgebildete handwurzel- und handgelenkumschließende Bandage ohne die Möglichkeit, die Hand in der medizinischen Funktionsstellung zu halten, ist der US-A-4,584,993 zu entnehmen. Diese Handgelenksbandage ermöglicht in keiner Weise eine Fixierung der Hand in der medizinischen Funktionsstellung. Die Grundgelenke der Langfinger sind nicht in einer 70°- bis 80°-Flexionsstellung lagerbar. Die Langfinger sind nach angelegter Handgelenksbandage frei beweglich.

Neben Handgelenkbandagen zur wahlweisen Anwendung als elastische Stütze oder zur Stillegung des Handgelenks nach DE-U-81 28 832.8 oder Handgelenkstützen mit einer handwurzel- und handgelenkumschließenden Bandage nach DE-A-3 910 318 oder nach DE-U-88 11 364.7, bei denen ausschließlich eine Stützung des Handgelenks vermittels einer volaren oder einer dorsalen Schiene erfolgt und die bei Luxationen und Distorsionen sowie bei Arthrosen der Handwurzel zur funktionellen bedingten Ruhigstellung eingesetzt werden, sind Arm- und Handgelenkstützen und -schienen in den verschiedensten Ausführungformen bekannt.

Die Orthese für Unterarm und Hand zur Entspannung und Ruhigstellung der Muskelansätze am Epicondylus radialis nach DE-U-91 12 665.7 ist in der Weise ausgebildet, daß eine Schiene den Unterarm und die Hand volar und/oder dorsal aufnimmt, die im Bereich des Handgelenkes eine Beugung von etwa 5° bis 10° aufweist, wobei die Schiene im Abstand distal zur Ellenbeuge endet. Über Gurte sind Unterarm und Hand an dieser Schiene fixierbar. Mit dieser Orthese soll eine entspannte leichte Beugestellung im Handgelenk und den Fingergelenken ermöglicht und eine Ruhigstellung der für den Schmerz verantwortlichen Muskelansätze gewährleistet werden.

Nach der EP-A-0 039 323 ist eine Schiene zur Reposition und Ruhigstellung von Finger- und Mittelhandfrakturen, sowie ein Verfahren zur Herstellung dieser Schiene bekannt. Diese Schiene besteht aus einem plastisch verformbaren Streifen, der an seinem Endbereich zumindest einen Querteil zur Fixierung am Vorderarm aufweist, wobei der Streifen an seinen beiden Längsrändern mit Schwächungen versehen ist, welche ein Verbiegen des Streifens in seiner Ebene ermöglichen. Dieser Streifen ist vorzugsweise zur Gänze mit einer Umhüllung aus nachgiebigem Material umgeben. Aufgrund dieser Ausgestaltung ist die Schiene leicht in die anatomisch richtige Form bringbar; sie behält jedoch die gewünschte Form bei. Um den Streifen in die anatomisch richtige Form zu bringen, ist dabei zu berücksichtigen, daß die Achsen der Finger von einer quer verlaufenden Handwölbung ausgehen und beim Abbiegen der Finger nicht mehr parallel verlaufen, wie bei der Streckstellung, sondern am Kahnbeinhöker zusammenlaufen. Die Fingerachsen verändern also, von einer Parallelstellung in der Streckstellung der Finger ausgehend, während des Abbiegens der Finger dauernd ihre Richtung bis sie letztlich bei vollständig abgebogenen Fingern auf den Kahnbeinhöker zielen.

Eine Ruhigstellung der eine Fraktur aufweisenden Finger kann nun nicht in der gestreckten Lage der Finger erfolgen, sondern muß in seiner mittleren Beugestellung der Finger vorgenommen werden, damit die Sehnen gestreckt sind und die Finger nicht versteifen. Dies bedingt, daß auch die Fingerschiene in eine der jeweiligen mittleren Beugestellungen des ruhig zu stellenden Fingers entsprechende Lage gebracht werden muß. Diese Schiene berücksichtigt diese anatomischen Gegebenheiten und ermöglicht ein Verbiegen des Streifens in seiner Ebene, wobei die Schwächungen des Streifens an seinen beiden Längsrändern es ermöglichen, daß der Streifen nicht nur in seiner Ebene verbogen und um seine Längsachse verdreht werden kann, sondern auch quer zur Längsachse in seiner Ebene verbogen werden kann, so daß dann die Längsachse eine in zwei im wesentlichen zueinander verdrehten Ebenen gekrümmte Kurve darstellt, wodurch sichergestellt werden soll, daß die Schiene für jeden Finger und jeden Bruch exakt in eine Lage bringbar ist, die der anatomischen Form und Lage des Fingers angepaßt ist.

Zur Behandlung von entzündlichen Sehnenerkrankungen, bei denen vorwiegend die Sehnenscheiden und das Gleitgewebe betroffen sind, werden auch Schienen eingesetzt, so u. a. auch eine Schiene, die therapeutisch zwischen einem Gipsverband und den bekannten Handgelenkbandagen mit stabilisierenden Elementen einzuordnen ist. Auf der einen Seite hat diese Schiene nicht die stabilisierende Wirkung eines Gipsverbandes und kann nicht zur Frakturbehandlung eingesetzt werden. Auf der anderen Seite stellt sie bei schmerzhaften Reizzuständen der Sehnenscheiden eine Überversorgung dar, zumal die entsprechenden Bandagen viel weniger auftragen und einen wesentlich höheren Tragekomfort bieten. Während leichte Reizzustände an den Sehnenscheiden mit stabilisierenden Handgelenkbandagen therapiert werden, steht zur Behandlung einer "richtigen" Sehnenscheidenentzündung kein geeignetes Hilfsmittel zur Verfügung. Üblicherweise werden diese Fälle mit einem Gipsverband oder einem halbstarren Stützverband in Funktionsstellung ruhiggestellt, d. h. die Stellung der Hand und die Stellung des Handgelenkes werden miteinander verbunden.

Aufgabe der vorliegenden Erfindung ist es, eine gattungsgemäße formbeständige Hand- und Handgelenkorthese, insbesondere zur Behandlung von Tendovaginitis, zu schaffen, bei der das Handgelenk die zur Mittelhand und zu den Fingergrundgliedern gehörenden Gelenke (Metacarpophalangialgelenke) und die dem zentralen Teil zu gelegenen und zwischen den Fingergliedern liegenden Gelenke (proximalen Interphalangialgelenke) mit von der Orthese umschlossen werden, bei der zur Ruhigstellung der Sehnenscheiden im Bereich des vorderen Abschlusses der Orthese eine zusätzliche Fixierung der Finger erfolgt und bei der die eingearbeitete Schiene die folgenden Winkelstellungen vorgibt:
- Handgelenk:: 40° zur Rückseite erfolgende Streckung (Dorsalextension)
- Metacarpophalangialgelenke:: 60° Beugung der Finger zur Handfläche hin (Palmarflexion)
- Proximale Interphalangialgelenke:: 60° Beugung der Finger zur Handfläche hin (Palmarflexion), wobei eine über einen längeren Zeitraum sich erstreckende Stabilisierung erreicht werden soll.

Diese Aufgabe wird durch die in Anspruch 1 genannten Merkmale gelöst.

Während herkömmliche Handgelenkorthesen im Bereich der Mittelhand enden, schließt eine derart erfindungsgemäß ausgebildete Orthese die Metacarpophalangialgelenke (MCP-Gelenke) und die proximalen Interphalangialgelenke (PIP-Gelenke) ein, wobei die Orthese diese Gelenke in Funktionshaltung fixiert. Zur Ruhigstellung der Sehnenscheiden ist am vorderen Abschluß des bandagenartigen Stützkörpers der Orthese zusätzlich eine Fixierung der Finger vorgesehen.

Der Stützkörper der Hand- und Handgelenkorthese ist formgenäht; sie nimmt die entsprechende Funktionshaltung ein und besteht aus unterpolsertem, steifem Gewebe. Um einen langen Hebel am Unterarm zu haben, verfügt die Orthese über einen langen Unterarm-Schaft. An der Hand reicht die Orthese entscheidend weiter als die bekannten Orthesen und Handgelenkstützen; sie endet gelenkübergreifend im Bereich der proximalen Phalanx. Der vordere Abschluß ist der anatomischen Kontur folgend abgeschrägt. Die Finger werden mit einem unelastischen Klettband fixiert. Dieses Klettband und zwei Fixierungspunkte befinden sich am vorderen Abschluß der Orthese.

Als stabilisierendes Element ist hohlhandseitig (volarseitig) eine löffelförmige, die Hohlhand abstützende und den Spitzgriff erlaubende Schiene integriert, die die gewünschten Winkel von 40° ± 5° eines rückseitig gelegenen Zuges in Längsrichtung (Dorsalextension) im Handgelenk sowie 60° ± 5° Beugung der Finger zur Handfläche hin (Palmarflexion) in den MCP- und PIP-Gelenken vorgibt. Diese Schiene befindet sich in einer Tasche mit Klettverschluß und kann jederzeit leicht herausgenommen werden, um ein Auswechseln gegen Schienen anderer Ausgestaltung vornehmen zu können. Damit ist eine abbauende Versorgung und eine Reinigung der Orthese möglich.

Die volarseitig positionierte Schiene weist außerdem eine gute Polsterung auf, um keinen Druck auf die hohlhandseitig gelegene Gefäße auszuüben, wobei diese Polsterung bevorzugterweise durch das Material erreicht wird, welches zur Herstellung der die Schiene aufnehmenden Tasche verwendet wird.

Die Orthese wird seitlich geschlossen. In die über die gesamte Länge der Orthese verlaufende Anziehöffnung ist im Bereich des Unterarmschaftes sowie der Vorhand ein textiler Einsatz aus bevorzugterweise hochelastischem Gewebe mit Daumenöffnung eingenäht, wodurch das Anziehen der Orthese erleichtert und eine geschlossene Optik und somit ein erhöhter Tragekomfort geschaffen wird. Die Orthese wird im Bereich des Unterarms sowie der Hand mit gegenläufig angeordneten breiten, unelastischen Verschlußbändern befestigt, die bevorzugterweise als Klettbänder ausgeführt sind, so daß eine Anpassung an unterschiedliche Armweiten und Handbreiten möglich ist.

Besonders vorteilhaft ist, daß die Orthese aufgrund ihrer konstruktiven Ausgestaltung die MCP- und PIP-Gelenke mit einschließt, wobei die Gelenke in der vorgegebenen Winkeleinstellung fixiert werden. Die vorderseitige Fixierung der Finger an der Orthese ermöglicht eine Ruhigstellung der Sehnenscheiden.

Als Tendovaginitisschiene ist die erfindungsgemäß ausgebildete Orthese dazu geeignet, den bekannterweise eingesetzten Dauerverband zur Behandlung der Sehnenscheidenentzündung zu ersetzen. Wie die Gipsversorgung ist auch die Orthese gelenkübergreifend im Bereich der proximalen Phalanx. Durch die besondere anatomische Situation, daß die Muskeln, die die Finger bewegen, im Unterarm sitzen und mit langen Sehnen mit den Gelenken verbunden sind, die alle durch das Nadelöhr Handgelenk verlaufen, ist es nicht ausreichend, zur Ruhigstellung der Sehnenscheiden ausschließlich das Handgelenk zu immobilisieren. Erst durch den Einschluß der Finger in die Orthese wird eine ausreichende Ruhigstellung erzielt. Der klassischen Gipsversorgung folgend immobilsiert die Tendovaginitis-Orthese Handgelenk und Hand in Funktionsstellung. Dabei wird das Handgelenk in 40° ± 5° Dorsalextension fixiert. Die MCP-Gelenke werden in 60° ± 5° Palmarflexion gebracht, ebenso auch die PIP-Gelenke in gleicher Weise wie die MCP-Gelenke.

Die Orthese wird eingesetzt bevorzugterweise zur Behandlung von Tendovaginitis, jedoch ist auch ein Einsatz bei schweren Distorsionen, nach Handgelenkoperationen, Sehnenoperationen, Radiusfrakturen und nach Abnahme von Gipsverbänden möglich, d. h. der Anwendungsbereich der Orthese erstreckt sich auch für alle Nachbehandlungen nach chirurgischen Eingriffen. Auch bei akuten Reizzuständen infolge von Navikular-Pseudoarthrose, Lunatummalazie und Arthrosen ist die Orthese erfolgreich einsetzbar.

Die Orthese ist therapeutisch ausgewogen ausgebildet und bringt Handgelenk und Hand in die optimale Funktionsstellung. Das Handgelenk wird stabilisiert und immobilisiert. Die Sehnenscheiden werden ruhiggestellt. Die Bewegung der Finger wird ausgeschaltet. Außerdem ist die Orthese rormbeständig und über einen längeren Zeitraum gleichbleibend stabilisierend. Außerdem sitzt die Orthese gut und ist nach den individuellen anatomischen Anforderungen des Patienten formbar; sie paßt sich unterschiedlichen Unterarmweiten und Handbreiten an. Außerdem ist die Orthese fest am Unterarm verankert. Ein unerwünschter Druck auf die volarseitig verlaufenden Gefäße wird vermieden.

Die Orthese erbringt noch weitere folgende Vorteile: Die Orthese scheuert nicht, verursacht keine Druckstellen, trägt wenig auf, weist ein geringes Gewicht auf, ist hautfreundlich sowie luft- und wasserdampfdurchlässig. Eine unangenehme Wärmewirkung wird vermieden. Die Orthese ist darüber hinaus bei der Erstversorgung schnell und einfach an die anatomischen Gegebenheiten anpaßbar; sie läßt sich bequem anziehen, ist schmutzunempfindlich und läßt sich problemlos waschen.

Vorteilhafte Ausgestaltungen der Erfindung sind in den Unteransprüchen gekennzeichnet.

Ausführungsbeispiele der Erfindung werden nachstehend anhand der Zeichnungen näher erläutert. Es zeigen
- Fig. 1: in einer schaubildlichen Seitenansicht die a Hand und Handgelenk angelegte Orthese mit einem den Unterarm übergreifenden Schaft,
- Fig. 2: in einer Ansicht von oben die Orthese,
- Fig. 3: die Orthese in einer Ansicht von unten,
- Fig. 4: eine Seitenansicht der Orthese in mittels Verschlußbändern verschlossenem Zustand,
- Fig. 5: eine Seitenansicht der Orthese mit geöffneten Verschlußbändern,
- Fig. 6: einen waagerechten Schnitt durch die Orthese mit innenwandseitig ausgebildeter Tasche zur Aufnahme einer Schiene,
- Fig. 7: eine Ansicht auf die Orthese von der Einschlupföffnung mit innenwandseitig angeordneter Tasche zur Aufnahme der Schiene,
- Fig. 8: eine schaubildliche Ansicht der Schiene,
- Fig. 9: eine Draufsicht auf die Schiene gemäß Fig. 8,
- Fig. 10: einen senkrechten Querschnitt gemäß Linie X-X in Fig. 9,
- Fig. 11: einen schematischen Längsschnitt durch die Schiene,
- Fig. 12: eine schematische schaubildliche Ansicht der Schiene,
- Fig. 13: eine Ansicht von oben auf die Schiene mit ihren unterschiedlichen Knickpunkten zur Abwinkelung einzelner Schienenabschnitte,
- Fig. 14: eine Ansicht der rechten Hand mit Hand- und Fingerknochen von dorsal.
- Fig. 15: eine schaubildliche, in Richtung vom unterarmseitigen Ende zur fingerseitigen Auflage verlaufende Ansicht einer weiteren Ausführungsform der Orthese,
- Fig. 16: eine schaubildliche, in Richtung von der fingerseitigen Auflage zum unterarmseitigen Ende verlaufende Ansicht der Orthese gemäß Fig. 15,
- Fig. 17: die Orthese in einer Ansicht von oben,
- Fig. 18: die Orthese in einer Ansicht von unten und
- Fig. 19: einen senkrechten Längsschnitt gemäß Linie XIX-XIX in Fig. 17.

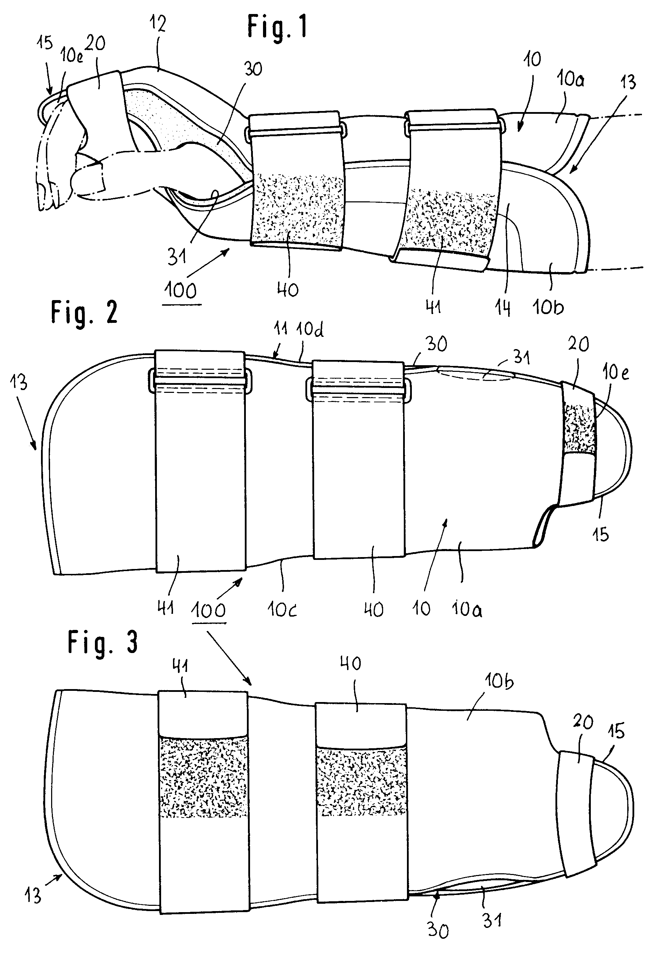

Die in den Fig. 1 bis 6 dargestellte Hand- und Handgelenkorthese 100 besteht aus einem formgenähten, Handgelenk und Hand hüllenartig umschließenden bandagenartigen Stützkörper 10 mit einem oberen Stützkörperteil 10a und einem unteren Stützkörperteil 10b. Die beiden Stützkörperteile 10a, 10b sind im Bereich der Stützkörperlängsseite 10c miteinander verbunden, während der Stützkörper 10 im Bereich seiner Längsseite 10d unter Ausbildung einer sich über die geamte Länge der Orthese erstreckenden Anziehöffnung offen ausgebildet ist, wobei jedoch diese Anziehöffnung 11 mittels eines textilen Einsatzes 30 verschlossen ausgebildet ist.

Die Fixierung der Orthese 100 am Unterarm, Handgelenk und Hand erfolgt mittels zweier, in ihren Längen veränderbaren Verschlußbändern 40, 41, bevorzugterweise aus unelastischem Material mit abwechselnd gegenläufigem oder gleichem Wicklungssinn. Diese Verschlußbänder 40, 41 sind zweckmäßigerweise als Klettbänder ausgebildet. Mittels der Verschlußbänder 40, 41 werden die beiden Stützkörperteile 10a, 10b des Stützkörpers 10 zusammengehalten, so daß im angelegten Zustand der Orthese 100 der Stützkörper 10 Handgelenk, Hand und Unterarm hüllenartig umschließt.

Einendseitig ist der Stützkörper 10 zur Ausbildung einer Einschlupföffnung 13 offen ausgebildet, so daß die Orthese über die Hand und den Unterarm gezogen werden kann.

Der Stützkörper 10 der Orthese 100 ist mit einem in etwa der Länge des Unterarmes entsprechenden Schaft 14 versehen, so daß die Orthese 100 im angelegten Zustand sich über den gesamten Unterarm erstreckt.

Der Stützkörper 10 ist an seinem der Einschlupföffnung 13 abgekehrten Ende mit einem fingergelenkübergreifenden Abschnitt 12 versehen, der im Bereich der proximalen Phalanx endet.

Der vordere Abschluß 15 des Stützkörpers 10 ist der anatomischen Kontur der Finger folgend abgeschrägt und mit einem unelastischen Band 20 versehen. Vermittels dieses Bandes 20 werden die Finger fixiert, wobei sich das Band 20 mit seinen Fixierungspunkten am vorderen Abschluß 10e der Orthese befindet (Fig. 2). Dieses unelastische Band 20 zur Fixierung der Finger weist nach der einen Ausführungsform eine vorgegebene, unveränderbare Länge auf, wobei jedoch auch nach einer weiteren Ausführungsform dieses Band 20 längenveränderbar ausgebildet ist. Die beiden freien Enden eines derart ausgebildeten Bandes 20 sind dann bevorzugterweise mittels eines Klettverschlusses verschließbar. Randabnäher am Band 20 verhindern ein Randeindrücken des Bandes auf die Fingeroberflächen.

Der die beiden Stützkörperteile 10a, 10b im Längsseitenbereich 10d verbindende textile Einsatz 30 besteht aus einem Gewebe mit eingenähter Daumenöffnung 31 (Fig. 1). Das Gewebe für diesen textilen Einsatz kann starr, jedoch auch elastisch ausgebildet sein. Das Fingerfixierungsband 20 sollte bevorzugterweise aus einem unelastischen Gewebe bestehen.

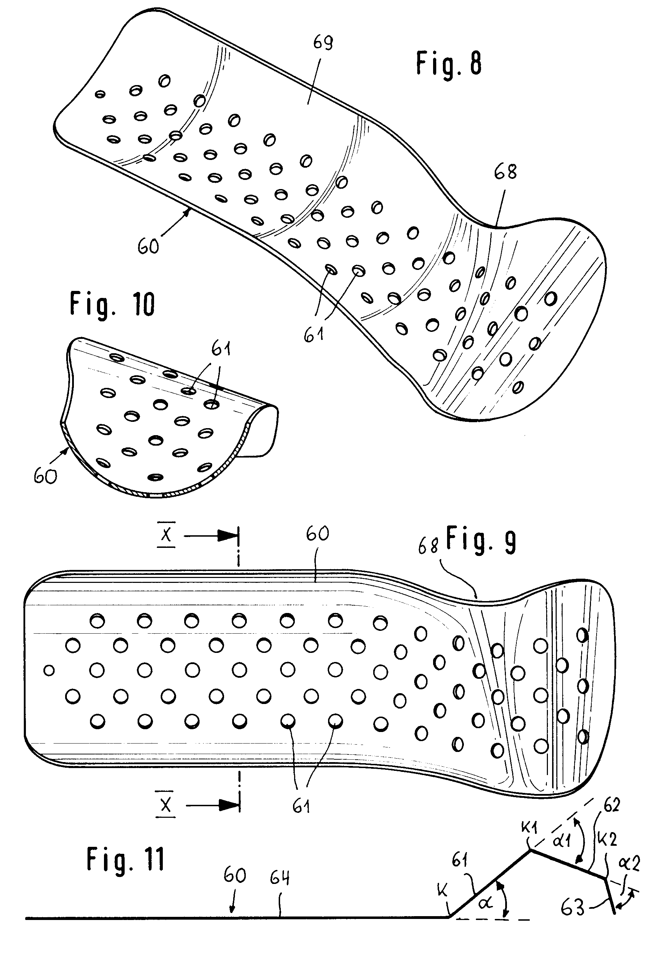

Der Stützkörper 10 der Orthese 100 ist mit einer volarseitig an der Innenwandfläche des Stützkörpers angeordneten, löffelförmigen, die Hohlhand abstützende und den Spitzgriff ermöglichende Schiene 60 versehen. Diese Schiene 60 besteht aus eine hohe Eigensteifigkeit aufweisendem Material, das nach erfolgter Formgebung nicht mehr nachträglich verformt werden kann. Bevorzugterweise besteht diese Schiene 60 aus metallischem Material, wobei auch andere geeignete Materialien eingesetzt werden können. Zur Verbesserung der Atmungsaktivität und zur Vermeidung eines Wärmestaus im Innenraum der angelegten Orthese ist diese Schiene 60 als Lochschiene mit einer Lochung 61 versehen (Fig. 8, 9 und 10).

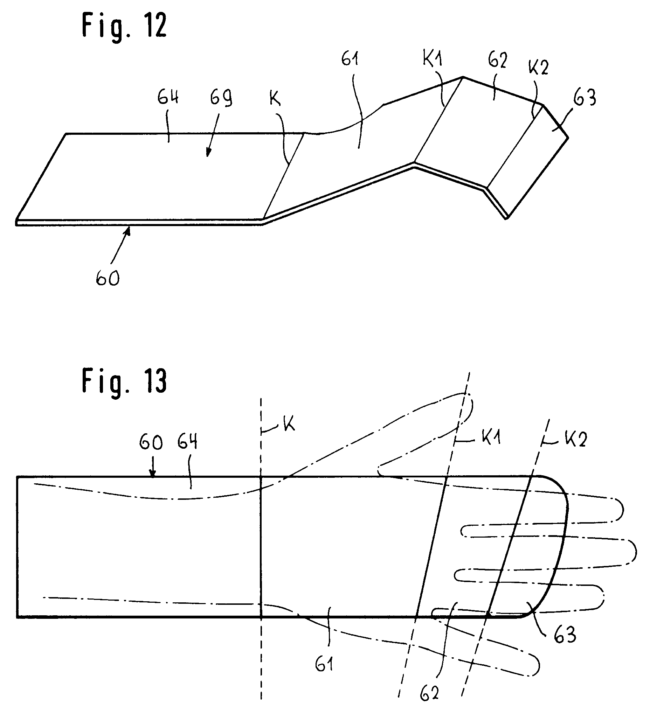

Die Schiene 60 weist im Bereich der Fingerglieder und der Mittelhandknochen drei zueinander abgebogene Schienenabschnitte 61, 62, 63 auf (Fig. 11 und 12) und im zweckmäßigerweise im Übergangsbereich vom Schienenschaft 69 zum Schienenabschnitt 61 eine seitliche Einziehung 68 für eine seitliche Daumenführung (Fig. 8 und 12).

Diese drei zueinander abgebogenen Schienenabschnitte 61, 62, 63 weisen zu dem längeren Schienenabschnitt 64 derartige Winkelstellungen auf, daß ein Winkel α zwischen den Schienenabschnitten 64, 61 40° ± 5° beträgt. Der Winkel α1 zwischen den Schienenabschnitten 61, 62 beträgt einen Winkel von 60° ± 5° und der Winkel α2 zwischen den Schienenabschnitten 62, 63 ebenfalls 60° ± 5°. Aufgrund dieser Winkelstellungen der einzelnen Schienenabschnitte zueinander werden die gewünschten Winkel von 40° Dorsalextension im Handgelenk HG sowie 60° Palmarflexion in den MCP- und PIP-Gelenken vorgegeben.

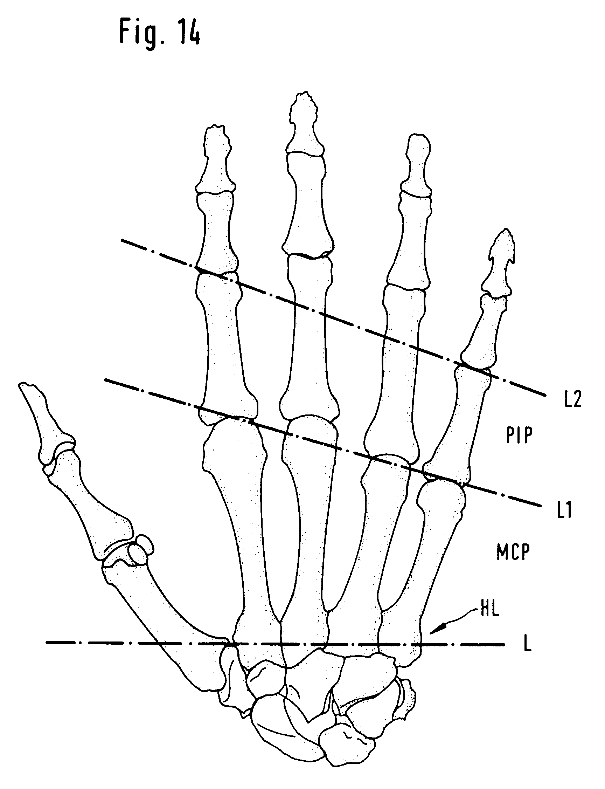

Da die MCP- und PIP-Gelenke der Hand nicht quer zur Unterarmlängsrichtung, sondern schrägverlaufend sind (Fig. 13 und 14), sind die Knicklinien K1, K2 zwischen den Schienenabschnitten 61, 62 und 62, 63 ebenfalls schrägverlaufend ausgebildet, wohingegen die Knicklinie K zwischen den Schienenabschnitten 64, 61 der Schiene 60 quer zur Schienenlängsrichtung verlaufend ist. Die zueinander abgewinkelten Abschnitte 64, 61, 62, 63 der Schiene 60 weisen hiernach in den quer zur Schienenlängsrichtung verlaufenden Knicklinien K, K1, K2 unterschiedliche Winkelstellungen, und zwar derart auf, daß die Knicklinie K im Bereich des Handgelenkes HG quer zur Schienenlängsrichtung verläuft. Die im Bereich der MCP-Gelenke liegende Knicklinie K1 der Schiene 60 verläuft entsprechend der durch die MCP-Gelenke verlaufenden schrägen Gelenklinie L1, während die im Bereich der PIP-Gelenke liegende Knicklinie K2 durch die Gelenklinie L2 verläuft, die durch die PIP-Gelenke vorgegeben ist (Fig. 13 und 14). Auf diese Weise ist die Schiene 60 den anatomischen Vorgaben der Hand angepaßt.

Der Stützkörper 10 der Orthese 100 besteht zweckmäßigerweise aus einem stark gepolsterten, steifen Gewebe, das außenseitig mit einer abwaschbaren Beschichtung oder Imprägnierung versehen sein kann.

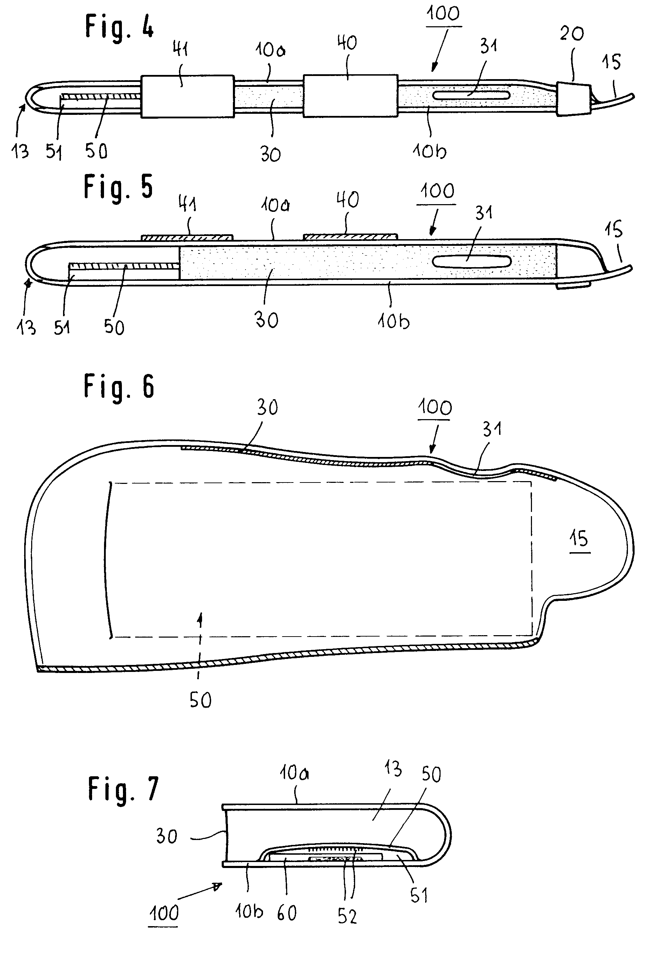

Die Schiene 60 ist an einer der beiden Innenwandflächen des Stützkörpers 10 gehalten. Hierzu ist an der Innenwandfläche des Stützkörpers 10 eine Tasche 50 vorgesehen, deren Schieneneinschuböffnung 51 mittels eine Verschlusses, wie z. B. Klettverschluß 52, verschließbar ist (Fig. 4, 5, 6 und 7). Bevorzugterweise ist die Tasche 50 für die Schiene 60 an der Innenwandfläche des Stützkörperteiles 10b befestigt (Fig. 4 und 5). Die Tasche 50 besteht aus einem Gewebezuschnitt, der mit dem Stützkörperteil 10b über eine Näh-, Klebe- oder Schweißverbindung verbunden ist. Eine Schweißverbindung wird immer dann Anwendung finden, wenn z. B. die miteinander zu verbindenden Teile ganz oder teilweise aus einer Kunststoffolie, einem Gewebe aus Kunststoffäden oder aus einem mit einem Kunststoff beschichteten oder imprägnierten Gewebe aus natürlichen Fasern bestehen. Die Schiene 60 kann zusätzlich mit einer Polsterung versehen sein, um keinen Druck auf die hohlhandseitig gelegenen Gefäße auszuüben. Auch der die Tasche 50 bildende Gewebezuschnitt kann aus einem gepolsterten Material bestehen.

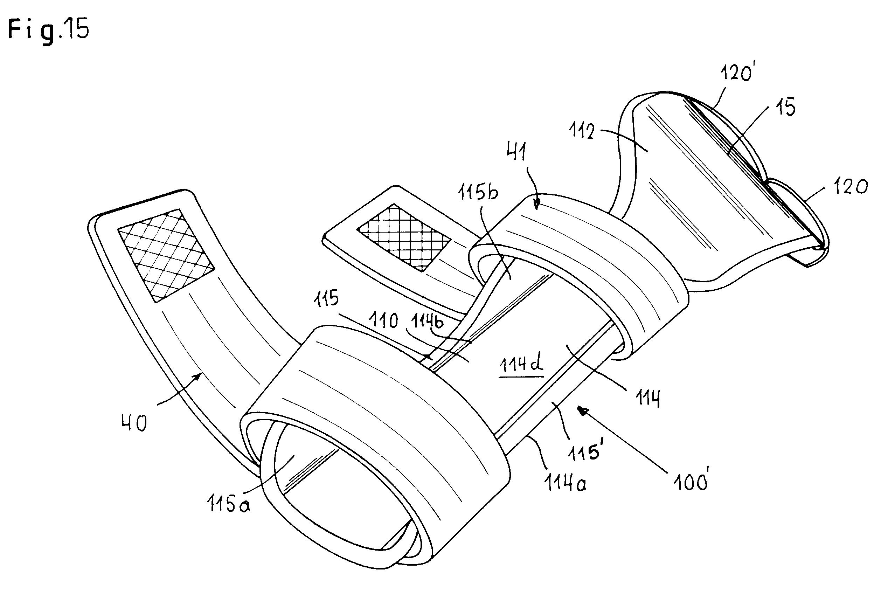

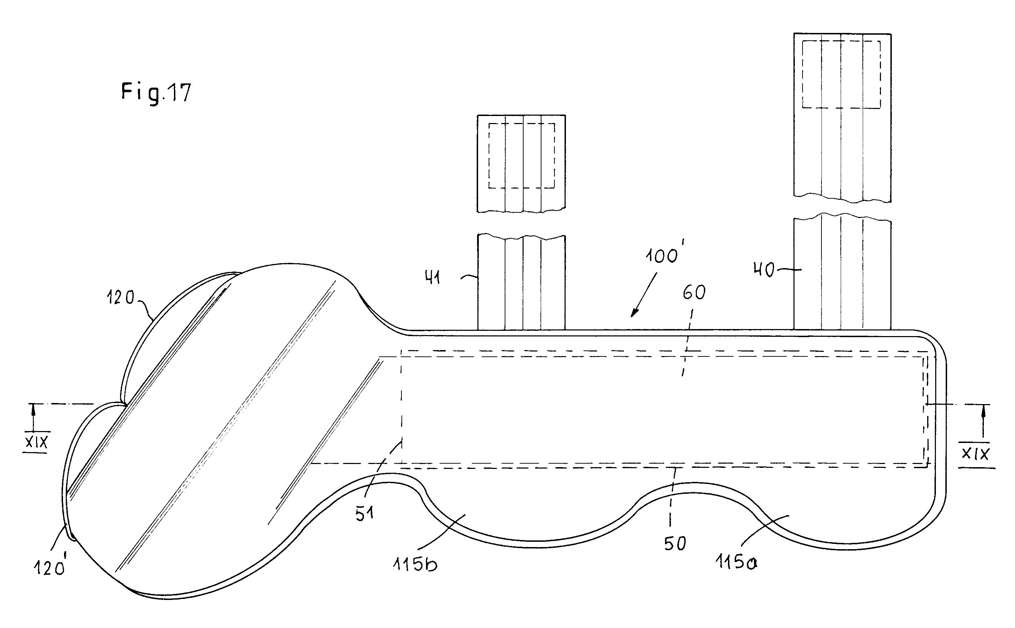

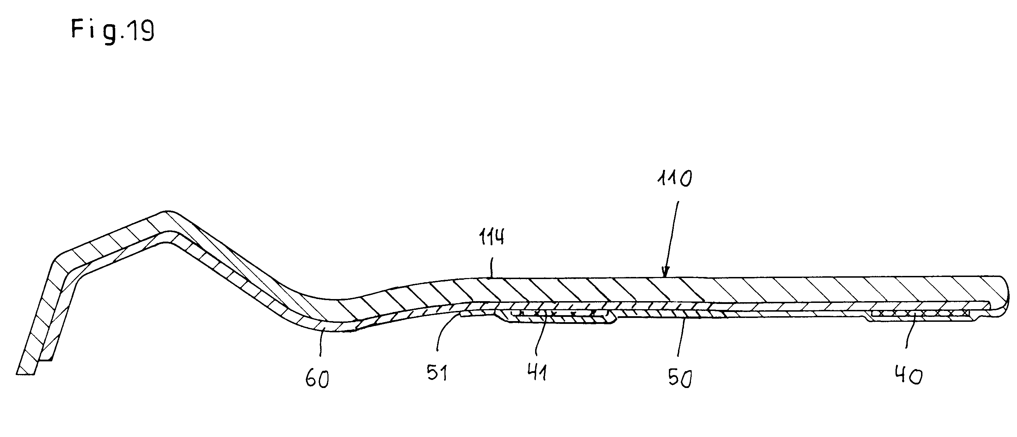

Die in Fig. 15 bis 19 gezeigte Ausführungsform der Hand-und Handgelenkorthese 100' weist in der Grundkonzeption eine der der Orthese 100 entsprechende Ausgestaltung auf.

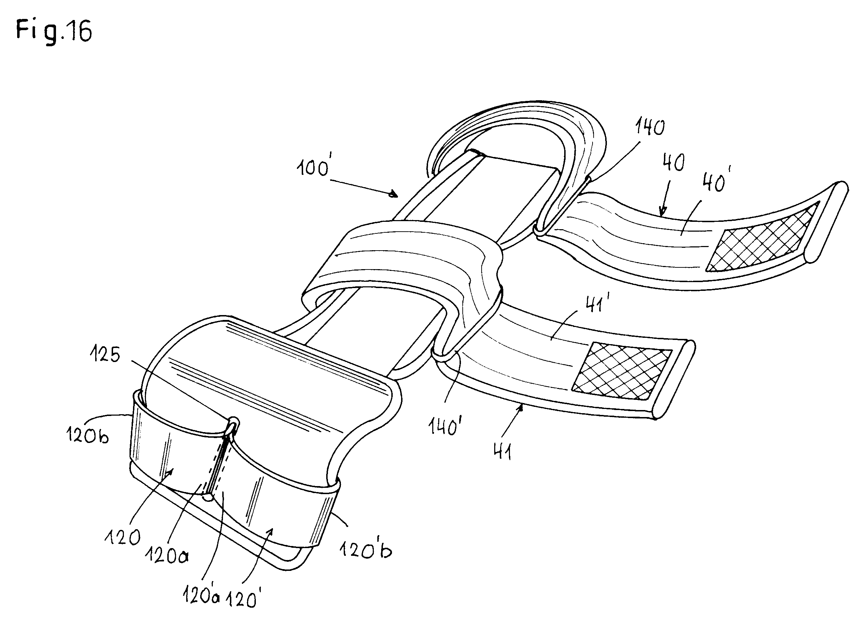

Bei der Orthese 100' wird der Stützkörper 110 von einem in etwa der Länge des Unterarms entsprechenden schaftartigen Zuschnitt 114 gebildet, so daß die Orthese 100' im angelegten Zustand sich über den gesamten Unterarm erstreckt. Einendseitig geht der schaftartige Zuschnitt 114 in einen fingergelenkübergreifenden Abschnitt 112 über, der im Bereich der proximalen Phalanx endet. Die Längsseiten 114a, 114b des schaftartigen Zuschnittes 114 weisen hochgezogene Randabschnitte 115, 115' auf, von denen der Randabschnitt 115 im Bereich der beiden Verschlußbänder 40, 41 mit lappenförmigen Verlängerungen bzw. Verbreiterungen 115a, 115b versehen ist, um eine seitliche Abstützung und Lagesicherung des Unterarms bei angelegter Orthese zu erhalten (Fig. 15 und 16).

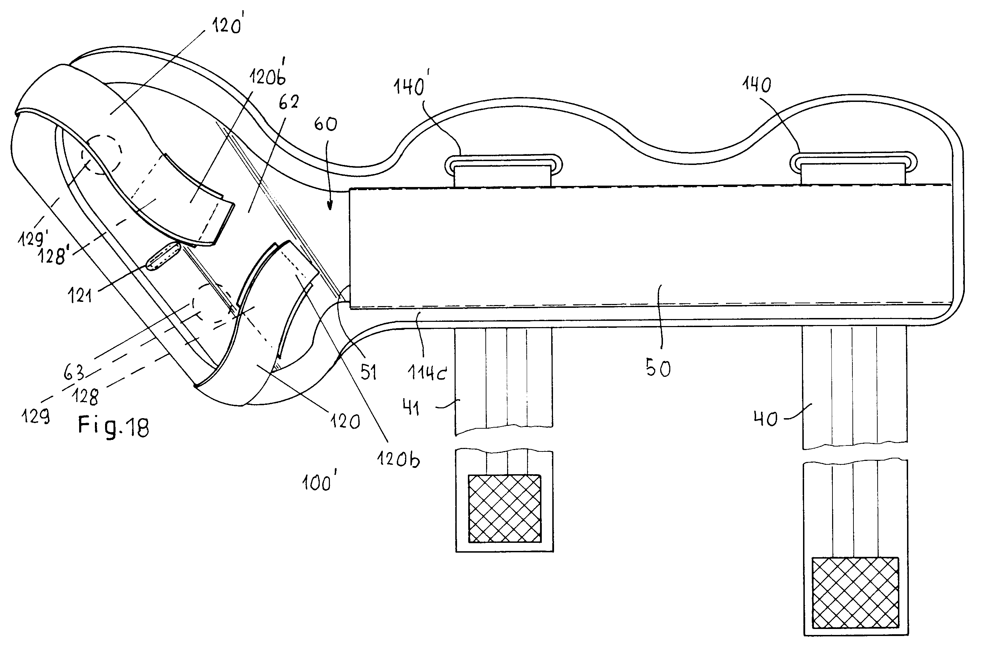

Die Rückseite 114c des Zuschnittes 114 weist eine mit einer dem fingergelenkübergreifenden Abschnitt 112 zugekehrten Schieneneinschuböffnung 51 versehenen Tasche 50 auf, die zur Aufnahme der Schiene 60 dient. Die Auflagefläche 114d des Zuschnittes 114 für den Unterarm und die Fingergelenke ist mit einem Zuschnitt aus einem gepolsterten Gewebe abgedeckt, das zweckmäßigerweise mit einer abwaschbaren Beschichtung oder Imprägnierung versehen ist oder aus einem frotteeartigen Gewebe besteht. Am vorderen Abschluß 15 des Stützkörpers 110 bzw. am Ende seines fingergelenkübergreifenden Abschnittes 112 sind zwei unelastische Fingerhaltebänder 120, 120' angeordnet, die der anatomischen Kontur der Finger folgend schräg verlaufend sind (Fig. 16, 17 und 18), wobei die Enden 120a, 120'a durch einen Schlitz 125 im äußeren Abschnitt 63 der Schiene 60 hindurchgeführt und auf der Rückseite der Schiene 60 an diesem Schienenabschnitt 63 vermittels einer Materialverdickung 121 oder einer anderweitig ausgebildeten Verstärkung fixiert sind (Fig. 18). Die anderen freien Enden 120b, 120'b der beiden Bänder 120, 120' sind über die äußeren Ränder des Schienenabschnittes 63 und des Schienenabschnittes 62 geführt und mittels Klettverbindungen 128, 128' an der Rückseite des Schienenabschnittes 62 lösbar befestigt, so daß über diese Klettverbindungen 128, 128' die Länge der beiden Bänder 120, 120' veränderbar und den jeweiligen Fingerstärken anpaßbar sind. Zweckmäßigerweise sind die beiden Klettverbindungen 128, 128' am Schienenabschnitt 62 vorgesehen (Fig. 18).

Der Stützkörper 110 weist eine gewisse Biegeelastizität auf; d. h., er besteht aus einem flexiblen, gepolsterten Gewebezuschnitt, der seine Steifigkeit durch die in die Tasche 50 eingeschobene Schiene 60 erhält. Ein Abheben des freien Endes des Zuschnittes 114 wird dadurch verhindert, daS das Zuschnittsende an der Außenseite des Schienenabschnittes 63 mittels einer oder zweier Klettverbindungen 129, 129' gehalten ist (Fig. 18).

Jedes Verschlußband 40, 41 besteht aus einer am Rand des Zuschnittes 114 befestigten Schnalle 140, 140' und einem bandförmigen Zuschnitt 40', 41', der am anderen Rand des Zuschnittes 114 befestigt ist. Im angelegten Zustand der Orthese werden die Verschlußbänder 40', 41' durch die Schnallen 140, 140' hindurchgeführt und so umgelegt, daß ihre Enden auf den Verschlußbandabschnitten zu liegen kommen, die zwischen dem Befestigungsbereich der Verschlußbänder 40', 41' und den Schnallen 140, 140' liegen. Die Enden der Verschlußbänder 40', 41' bilden zusammen mit dem Material der Verschlußbänder 40', 41' die Klettverschlüsse, wobei der eine Teil der Klettverschlüsse an den Verschlußbandenden ausgebildet ist, während der andere Teil der Klettverschlüsse vom Verschlußbandmaterial gebildet werden.

## Patentansprüche

1. Hand- und Handgelenkorthese, insbesondere zur Behandlung von Tendovaginitis, die aus einem an der Hand und an dem Handgelenk mittels Verschlußbändern gehaltenen, formgenähten Stützkörper mit einer volaren, die Hohlhand abstützenden, starren Schiene (60) besteht, um die Hand und die Finger in Funktionsstellung zu halten, und die mit einem fingerzugewandten Abschnitt (12) gelenkübergreifend im Bereich der Fingerglieder endet und sich über den größten Teil der Finger erstreckt, wobei die Schiene (60) im Bereich der Fingerglieder und der Mittelhandknochen drei zueinander abgebogene Schienenabschnitte (61,62,63) aufweist, die in teils quer und teils schräg zur Schienenlängsrichtung verlaufenden Knicklinien (K,K1,K2) Stellungen in unterschiedlichen Winkeln zueinander derart aufweisen, daß eine erste Knicklinie (K) im Bereich des Handgelenks (HG) quer zur Schienenlängsrichtung, die im Bereich der Mittelhandgelenke liegende zweite Knicklinie (K1) entsprechend der durch die Mittelhandgelenke gegebenen und zur Schienenlängsrichtung schräg verlaufenden Gelenklinie (L1) und die im Bereich der dem zentralen Bereich zu gelegenen Fingergliedergelenke liegende dritte Knicklinie (K2) entsprechend der durch diese Fingergliedergelenke verlaufende Gelenklinie (L2) schräg zur Schienenlängsrichtung verläuft, wobei die abgewinkelten Schienenabschnitte (61,62,63) mit der Lage der Gelenk-Knicklinien (L,L1,L2) zusammenfallen und der Winkel α zwischen den Schienenabschnitten (64,61) 40° ± 5°, der Winkel α1 zwischen den Schienenabschnitten (61, 62) 60° ± 5° und der Winkel α2 zwischen den Schienenabschnitten (62,63) 60° ± 5° beträgt.

2. Hand- und Handgelenkorthese nach Anspruch 1, dadurch gekennzeichnet, daß die Orthese (100) aus einem formgenähten Handgelenk und Hand hüllenartig umschließenden Stützkörper (10;110) aus einer volarseitig an der Innenwandfläche des Stützkörpers angeordneten, den Spitzgriff ermöglichenden löffelförmigen Schiene (60) und aus dem einseitig in Längsrichtung unter Ausbildung einer sich über die gesamte Länge der Orthese erstreckenden Anziehöffnung (11) offen ausgebildeten Stützkörper (10) mit zwei über verstellbare Verschlußbänder (40,41) zusammengehaltenen Stützkörperteilen (10a,10b) und mit einem der Länge des Unterarmes entsprechenden Schaft (14) besteht.

3. Hand- und Handgelenkorthese nach einem der Ansprüche 1 und 2, dadurch gekennzeichnet, daß der vordere Abschluß (15) des Stützkörpers (10) der anatomischen Kontur der Finger folgend so ausgebildet ist, daß der seitlich gelegene Bereich gegenüber dem in der Mitte liegenden Bereich weiter zurückliegend und abgeschrägt ist.

4. Hand- und Handgelenkorthese nach einem der Ansprüche 1 bis 3, dadurch gekennzeichnet, daß die Finger mittels eines unelastischen Bandes (20) fixiert sind, das sich mit seinen Fixierungspunkten in einem Abstand zum vorderen Ende der Orthese (100) befindet.

5. Hand- und Handgelenkorthese nach einem der Ansprüche 2 bis 4, dadurch gekennzeichnet, daß im Bereich des Unterarmschaftes (14) des Stützkörpers (10) und der Vorhand ein textiler, die beiden Stützkörperteile (10a,10b) verbindender, sich über die gesamte Länge oder über einen Teil hiervon des Stützkörpers (10) erstreckender Einsatz (30) aus einem Gewebe mit eingenähter Daumenöffnung (31) vorgesehen ist.

6. Hand- und Handgelenkorthese nach einem der Ansprüche 1 bis 5, dadurch gekennzeichnet, daß der Stützkörper (10) aus einem unterpolsterten, steifen Gewebe besteht.

7. Hand- und Handgelenkorthese nach Anspruch 5, dadurch gekennzeichnet, daß der die beiden Stützkörperteile (10a,10b) verbindende textile Einsatz (30) mit Daumenöffnung (31) aus einem hochelastischen Gewebe besteht.

8. Hand- und Handgelenkorthese nach einem der Ansprüche 2 bis 7, dadurch gekennzeichnet, daß die Orthese (100) im Bereich des Unterarms sowie der Hand mit breiten, unelastischen Klettbändern als Verschlußbänder (40,41) mit abwechselnd gegenläufigem Wicklungssinn versehen ist.

9. Hand- und Handgelenkorthese nach einem der Ansprüche 1 bis 8, dadurch gekennzeichnet, daß für die Anordnung der starren Schiene (60) an der Innenwandfläche des Stützkörpers (10) dieser innenwandseitig mit einer Tasche (50) versehen ist, deren Schieneneinschuböffnung (51) mittels eines Verschlusses, wie Klettverschluß (52), verschließbar ist.

10. Hand- und Handgelenkorthese nach einem der Ansprüche 1 bis 9, dadurch gekennzeichnet, daß der Stützkörper (110) aus einem in etwa der Länge des Unterarms entsprechenden schaftartigen Zuschnitt (114) besteht, der einendseitig in einen fingergelenkübergreifenden Abschnitt (112) übergeht, der im Bereich der proximalen Phalanx endet, wobei die Längsseiten (114a,114b) des Zuschnittes (114) hochgezogene Ränder bzw. Randabschnitte (115,115') aufweisen, von denen der Randabschnitt (115) im Bereich der beiden Verschlußbänder (40,41) mit lappenförmigen Verlängerungen bzw. Verbreiterungen (115a,115b) versehen sind, und daß der Stützkörper (110) auf seiner Rückseite (114c) eine mit einer Einschuböffnung (51) versehene Tasche (50) für die Schiene (60) aufweist.

11. Hand- und Handgelenkorthese nach Anspruch 10, dadurch gekennzeichnet, daß am vorderen Abschluß (15) des Stützkörpers (110) zwei unelastische Fingerhaltebänder (120,120') angeordnet sind, die der anatomischen Kontur der Finger folgend schräg verlaufend sind und die mit ihren Enden (120a,120'a) durch einen Schlitz (125) im äußeren Abschnitt (63) der Schiene (60) hindurchgeführt und auf der Schienenrückseite an dem Schienenabschnitt (63) vermittels einer Materialverdickung (121) fixiert sind, wobei die anderen freien Enden (120b,120'b) der beiden Bänder (120,120') über die äußeren Ränder des Schienenabschnittes (63) und des Schienenabschnittes (62) geführt und mittels Klettverbindungen (128,128') an der Rückseite des Schienenabschnittes (62) lösbar befestigt sind.

12. Hand- und Handgelenkorthese nach einem der Ansprüche 10 und 11, dadurch gekennzeichnet, daß der Stützkörper (110) aus einem flexiblen, gepolsterten Gewebezuschnitt besteht, der seine Steifigkeit durch die in die Tasche (50) eingeschobene Schiene (60) erhält.

13. Hand- und Handgelenkorthese nach einem der Ansprüche 10 bis 11, dadurch gekennzeichnet, daß das freie Ende des fingergelenkübergreifenden Abschnittes (112) des Zuschnittes (114) des Stützkörpers (110) an der Aussenseite des Schienenabschnittes (63) mittels einer oder zweier Klettverschlüsse (129,129') gehalten ist.

14. Hand- und Handgelenkorthese nach einem der Ansprüche 10 bis 13, dadurch gekennzeichnet, daß jedes Verschlußband (40,41) aus einer am Rand des Zuschnittes (114) befestigten Schnalle (140;140') und einem bandförmigen Zuschnitt (40';41') besteht, wobei im angelegten Zustand der Orthese (100') die bandförmigen Zuschnitte (40',41') durch die Schnallen (140, 140') hindurchgeführt und so umgelegt sind, daß die freien Enden der bandförmigen Zuschnitte (40',41') auf den Zuschnittsabschnitten zu liegen kommen, die zwischen dem Befestigungsbereich der bandförmigen Zuschnitte (40',41') und den Schnallen (140,140') liegen, wobei die Enden der bandförmigen Zuschnitte (40',41') an diesen mittels Klettverschlüssen gehalten sind.

## Claims

1. Hand and wrist orthosis, more particularly for the treatment of tenosynovitis, which is comprised of a true-to-shape-sewn supporting member with a rigid splint (60) on the volar side supporting the hollow hand, said supporting member being retained on the hand and on the wrist with the aid of fastening tapes in order to maintain the hand and the fingers in the functional position and which, with a section (12) facing the fingers, terminates so as to engage over the joints within the area of the phalanxes and extends over the greatest part of the fingers, wherein the splint (60), within the region of the phalanxes and the metacarpal bones possesses three splint sections (61,62,63) bent off towards each other, which, in the kink lines (K, K1, K2) proceeding partly transversely and partly obliquely to the longitudinal direction of the splint, assume positions at different angles in relation to each other in such a way that a first kink line (K), within the region of the wrist (HG), proceeds transversely to the longitudinal direction of the splint, the second kink line (K1) located within the region of the metacarpal joints, in accordance with the joint line (L1) provided by the metacarpal joints and proceeding obliquely to the longitudinal direction of the splint and the third kink line (K2) located within the region of the phalangeal joints located in the direction of the central area, in accordance with the joint line (L2) proceeding through these phalangeal joints, proceeds obliquely to the longitudinal direction of the splint, in which case the angled splint sections (61,62,63) coincide with the position of the joint kink lines (L, L1, L2) and the angle α between the splint sections (64,61) is 40° ± 5°, the angle α1 between the splint sections (61,62) is 60° ± 5° and the angle α2 between the splint sections (62,63) is 60° ± 5°.

2. Hand and wrist orthosis according to Claim 1,
characterized in that
the orthosis (100) is comprised of a supporting member (10;110) that is sewn true to shape and ensheathingly enclosing wrist and hand, of a spoon-shaped splint (60) disposed on the volar side on the inner all area of the supporting member, making the pointed gripping possible and of the openly constructed supporting member (10) extending on one side in the longitudinal direction while forming a putting-on or pulling-on aperture (11) extending over the entire length of the orthosis, with two supporting member portions (10a,10b) held together by means of adjustable fastening tapes (40,41) and with a shaft (14) corresponding to the length of the forearm.

3. Head and wrist orthosis according to either Claim 1 or 2,
characterized in that
the front termination (15) of the supporting member (10), while following the anatomical contour of the fingers, is constructed in such a way that the laterally located area, relative to the area situated in the center, lies further back and is sloped.

4. Hand and wrist orthosis according to any of Claims 1 to 3,
characterized in that
the fingers are fixated with the aid of an elastic tape (20), which, with its points of fixation, is disposed so as to be spaced away from the front end of the orthosis (100).

5. Hand and wrist orthosis according to any of Claims 2 to 4,
characterized in that,
within the area of the forearm shaft (14), the supporting member (10) and the forehand, a textile inset (30) interconnecting the two supporting member portions (10a,10b) and extending over the entire length or over a part hereof of the supporting member (10) of a fabric with sewn-in thumb aperture (31) is provided.

6. Hand and wrist orthosis according to any of Claims 1 to 5,
characterized in that
the supporting member (10) is comprised of a padded, rigid fabric.

7. Hand and wrist orthosis according to Claim 5,
characterized in that
the textile inset (30) with the thumb aperture (31) interconnecting the two supporting member portions (10a,10b) is comprised of a highly elastic fabric.

8. Hand and wrist orthosis according to any of Claims 2 to 7,
characterized in that
the orthosis (100), within the area of the forearm as wll as that of the hand, is provided with wide, inelastic Velcro tapes in the form of fastening tapes (40,41) with alternate right and left inding direction.

9. Hand and wrist orthosis according to any of Claims 1 to 8,
characterized in that,
for the disposition of the rigid splint (60) on the inner wall area of the supporting member (10), the same is provided on the side of the inner wall with a pocket (50), whose splint insertion aperture (51) can be closed with the aid of a fastening means, such as a Velcro fastener (52).

10. Hand and wrist orthosis according to any of Claims 1 to 9,
characterized in that
the supporting member (110) is comprised of a shaft-like blank (114) corresponding approximately to the length of the forearm, which, at one end, passes into a section (112) engaging over the phalanxes, which terminates in the region of the proximal phalanx, in which case the longitudinal sides (114a, 114b) of the blank (114) possess raised borders or border sections (115,115'), of which the border section (115) within the region of the two fastening tapes (40,41) are provided with tab-like extensions or widened portions (115a,115b) and in that the supporting member (110), on its rear (114c), possesses a pocket (50) provided with an insertion aperture (51) for the splint (60).

11. Hand and wrist orthosis according to Claim 10,
characterized in that,
at the front termination (15) of the supporting member (110), two inelastic finger retaining straps (120,120') are disposed which, while following the anatomical contour of the fingers, proceed obliquely and which, with their ends (120a,120'a), are passed through a slot (125) in the outer section (63) of the splint (60) and, on the rear of the splint, are fixed to the splint section (63) with the aid of a thickened material portion (121), while the other free ends (120b, 120'b) of the two straps (120,120') are carried across the other borders of the splint section (62) and, with the aid of Velcro tape connections (128,128'), are detachably fixed to the rear of the splint section (62).

12. Hand and wrist orthosis according to either Claim 10 or 11,
characterized in that
the supporting member (110) is comprised of a flexible, padded fabric blank which receives its rigidity from the splint (60) inserted into the pocket (50).

13. Hand and wrist orthosis according to any of Claims 10 to 13,
characterized in that
the free end of the section (112) engaging over the phalanxes of the blank (114) of the supporting member (110) is, on the outside of the splint section (63), retained with the aid of one or two Velcro fasteners (129,129').

14. Hand or wrist orthosis according to any of Claims 10 to 13,
characterized in that
each fastening tape (40,41) is comprised of a buckle (140;140') attached to the border of the blank (114) and of a tape-like blank (40';41'), in which case, in the applied state of the orthosis (100'), the tape-like blanks (40',41') are passed through the buckles (140,140') and are turned down in such a way that the free ends of the tape-like blanks (40', 41') come to lie upon the blank sections that are located between the attachment area of the tape-like blanks (40',41') and the buckles (140.140'), while the ends of the tape-like blanks (40',41') are retained on the same with the aid of Velcro fasteners.

## Revendications

1. Orthèse pour main et poignet, en particulier pour le traitement de tendinite, qui est constituée par un corps d'appui cousu en forme, maintenu au poignet au moyen de bandes de fermeture, avec une attelle (60) palmaire rigide, qui soutient la paume de la main, pour maintenir la main et les doigts en position fonctionnelle et qui se termine avec une section tournée vers les doigts (12) en recouvrant les articulations dans la zone des doigts et qui s'étend sur la plus grande partie des doigts, l'attelle (60) présentant, dans la zone des doigts et des os de la partie médiane de la main, trois sections d'attelle (61, 62, 63) courbées l'une vers l'autre qui présentent, dans les lignes d'inflexion (K, K1, K2) qui sont en partie transversales et en partie en biais par rapport au sens longitudinal de l'attelle, des positions dans différents angles l'une par rapport à l'autre de telle manière qu'une première ligne d'inflexion (K) dans la zone du poignet (HG) est transversale par rapport au sens longitudinal de l'attelle, la seconde ligne d'inflexion (K1) qui se situe dans la zone des articulations de la partie médiane de la main va selon la ligne d'articulations (L1) qui est donnée par les articulations de la partie médiane de la main et qui est en biais par rapport au sens longitudinal de l'attelle et la troisième ligne d'inflexion (K2), qui se situe dans la zone des articulations des doigts situées vers la zone centrale est en biais selon la ligne d'articulations (L2) qui passe par ces articulations de doigts, les sections d'attelle coudées (61, 62, 63) coïncidant avec la position des lignes d'inflexion des articulations (L, L1, L2) et l'angle α entre les sections d'attelle (64, 61) étant de 40 ± 5°, l'angle α1 entre les sections d'attelle (61, 62) étant de 60 ± 5° et l'angle α2 entre les sections d'attelle (62, 63) étant de 60 ± 5°.

2. Orthèse pour main et poignet selon la revendication 1, caractérisée en ce que l'orthèse (100) est constituée par un corps d'appui (10 ; 110) cousu en forme, entourant à la manière d'une enveloppe le poignet et la main, constitué par une attelle (60) en forme de cuiller placée du côté de la paume sur la face de la paroi intérieure du corps d'appui, permettant l'action de saisir en pointe, et par le corps d'appui (10), configuré ouvert sur un côté dans le sens longitudinal en formant une ouverture pour mettre l'orthèse (11) qui s'étend sur toute la longueur de l'orthèse, avec deux parties de corps d'appui (10a, 10b) maintenues ensemble par des bandes de fermeture (40, 41) réglables et avec une tige (14) correspondant à la longueur de l'avant-bras.

3. Orthèse pour main et poignet selon l'une des revendications 1 et 2, caractérisée en ce que la fermeture avant (15) du corps d'appui (10) est configurée en suivant le contour anatomique des doigts de telle manière que la zone située latéralement est plus rentrée par rapport à la zone située au milieu et est taillée en biseau.

4. Orthèse pour main et poignet selon l'une des revendications 1 à 3, caractérisée en ce que les doigts sont fixés au moyen d'une bande non élastique (20) qui se trouve avec ses points de fixation à un certain écart par rapport à l'extrémité antérieure de l'orthèse (100).

5. Orthèse pour main et poignet selon l'une des revendications 2 à 4, caractérisée en ce que, dans la zone de la tige de l'avant-bras (14) du corps d'appui (10) et de la partie antérieure de la main, un insert textile (30) est prévu qui relie les deux parties de corps d'appui (10a, 10b), qui s'étend sur toute la longueur ou sur une partie de la longueur du corps d'appui (10), insert qui est en un tissu avec une ouverture pour le pouce (31) cousue à l'intérieur.

6. Orthèse pour main et poignet selon l'une des revendications 1 à 5, caractérisée en ce que le corps d'appui (10) est constitué par un tissu rigide rembourré par en dessous.

7. Orthèse pour main et poignet selon la revendicatioon 5, caractérisée en ce que l'insert textile (30) avec l'ouverture pour le pouce (31), insert qui relie les deux parties de corps d'appui (10a, 10b), est constitué par un tissu très élastique.

8. Orthèse pour main et poignet selon l'une des revendications 2 à 7, caractérisée en ce que l'orthèse (100) est pourvue, dans la zone de l'avant-bras et de la main, de larges bandes auto-agrippantes non élastiques comme bandes de fermeture (40, 41) avec un sens d'enroulement en sens opposé en alternance.

9. Orthèse pour main et poignet selon l'une des revendications 1 à 8, caractérisée en ce que, pour la disposition de l'attelle rigide (60) sur la face de paroi intérieure du corps d'appui (10), celui-ci est pourvu, sur le côté de la paroi intérieure, d'une poche (50) dont l'ouverture pour insérer l'attelle (51) peut être fermée au moyen d'une fermeture, comme une fermeture auto-agrippante (52).

10. Orthèse pour main et poignet selon l'une des revendications 1 à 9, caractérisée en ce que le corps d'appui (110) est constitué par une pièce découpée du type tige (114) qui correspond à peu près à la longueur de l'avant-bras qui se poursuit, du côté de l'une des extrémités, en une section recouvrant les articulations des doigts (112) qui se termine dans la zone de la phalange proximale, les longs côtés (114a, 114b) de la pièce découpée (114) présentant des bords ou des sections de bord (115, 115') relevées, parmi lesquelles la zone de bord (115) dans la zone des deux bandes de fermeture (40, 41) est pourvue de prolongements ou d'élargissements en forme de pattes (115a, 115b), et que le corps d'appui (110) présente, sur sa face arrière (114c), une poche (50) pourvue d'une ouverture d'insertion (51) pour l'attelle (60).

11. Orthèse pour main et poignet selon la revendication 10, caractérisée en ce que deux bandes de maintien des doigts (120, 120'), non élastiques, sont placées sur l'arête avant (15) du corps d'appui (110), bandes qui vont en biais en suivant le contour anatomique des doigts et qui traversent avec leurs extrémités (120a, 120'a) une fente (125) dans la section extérieure (63) de l'attelle (60) et qui sont fixées au dos de l'attelle sur la section de l'attelle (63) au moyen d'un épaississement de matière (121), les autres extrémités libres (120b, 120'b) des deux bords (120, 120') passant sur les bords extérieurs de la section d'attelle (63) et de la section d'attelle (62) et étant fixées de manière amovible au dos de la section d'attelle (62) au moyen de raccords auto-agrippants (128, 128').

12. Orthèse pour main et poignet selon l'une des revendications 10 et 11, caractérisée en ce que le corps d'appui (110) est constitué par une pièce découpée en tissu flexible, rembourré qui obtient sa rigidité par l'attelle (60) insérée dans la poche (50).

13. Orthèse pour main et poignet selon l'une des revendications 10 à 11, caractérisée en ce que l'extrémité libre de la section qui recouvre les articulations des doigts (112) de la pièce découpée (114) du corps d'appui (110) est maintenue sur la face extérieure de la section d'attelle (63) au moyen d'une ou de deux fermetures auto-agrippantes (129, 129').

14. Orthèse pour main et poignet selon l'une des revendications 10 à 13, caractérisée en ce que chaque bande de fermeture (40, 41) est constituée par une boucle (140 ; 140') fixée au bord de la pièce découpée (114) et par une pièce découpée en forme de bande (40' ; 41'), les pièces découpées en forme de bande (40', 41') traversant les boucles (140, 140') et étant rabattues, lorsque l'orthèse (100') est à l'état appliqué, de telle manière que les extrémités libres des pièces découpées en forme de bande (40', 41') viennent reposer sur les sections de pièce découpée qui se situent entre la zone de fixation des pièces découpées en forme de bande (40', 41') et les boucles (140, 140'), les extrémités des pièces découpées en forme de bande (40', 41') étant maintenues sur celles-ci au moyen de fermetures auto-agrippantes.
